# EUROPEAN PATENT APPLICATION

(11) **EP 1 258 253 A1**
(43) Date of publication of application: **20.11.2002**
(21) Application number: 01901552.8
(22) Date of filing: 26.01.2001
(51) Int. Cl.: A61K 45/06, A61K 31/403, A61K 31/343, A61K 31/381, A61K 31/445, A61K 31/216, A61K 31/22, A61K 31/337, A61K 31/18, A61K 31/702, A61K 31/195, A61K 38/28, A61K 31/426, A61P 43/00, A61P 13/02, A61P 3/10, A61P 3/06, C07D 209/88

(54) **NOVEL REMEDIES WITH THE USE OF BETA3 AGONIST**

(30) Priority: 28.01.2000 JP 2000020733
(71) Applicant: Asahi Kasei Kabushiki Kaisha, Osaka-shi, Osaka 530-8205 (JP)
(72) Inventor: OGAWA, Kohei, Mishima-shi, Shizuoka 411-0802 (JP); UMENO, Hiroshi, Kannami-cho, Tagata-gun, Shizuoka 419-01 (JP)
(74) Representative: Forstmeyer, Dietmar, Dr. rer. nat., Dipl.-Chem.
(86) International application number: JP0100553
(87) International publication number: WO01054728

(57) **Abstract**

Provided is a therapeutic agent comprising at least a compound having a β3 agonist activity and one member selected from the group consisting of an anticholinergic agent, a monoamine reuptake inhibitor, a lipase inhibitor, a selective serotonin reuptake inhibitor, insulin, an insulin secretagogue, biguanide, an α-glucosidase inhibitor, an insulin sensitizer, a HMG-CoA reductase inhibitor, an anion exchange resin, a fibric acid derivative and a nicotinic acid derivative. The β3-agonist has an activity of inhibiting urinary incontinence. Further, when used together with a remedy for urinary incontinence such as propiverine, oxybutynin hydrochloride or tolterodine, it exerts an enhanced anti-urinary incontinence effect. When used together with an antiobestic agent such as sibutramine or orlistat, it exerts an enhanced antiobestic effect. When used together with an antidiabetic agent such as insulin, glibenclamide, acarbose or rosiglitazone, it exerts an enhanced antidiabetic effect. When used together with an antilipemic agent such as bezafibrate or pravastatin, it exerts an enhanced antilipemic effect.

## Description

### FIELD OF THE INVENTION

The present invention relates to novel therapeutic agents that use a β3 agonist.

### BACKGROUND OF THE INVENTION

β adrenaline receptors are classified into β1, β2, and β3. It is considered that β1 stimulation increases the heart rate, β2 stimulation induces relaxation of smooth muscle tissue and reduces the blood pressure, and β3 promotes lipolysis of adipocytes and increases thermogenesis. Accordingly, it is shown that a β3 agonist is useful as a therapeutic agent for diabetes, obesity and prevention of hyperlipidemia (Nature 309, p163-165 (1984); Int. J. Obes. Relat. Metab. Disord. 20, p191-199 (1996); Drug Development Research 32, p69-76 (1994); J. Clin. Invest. 101, p2387-2393 (1998)).

Recently, it has been shown that β3 adrenaline receptors are expressed in the detrusor muscle, and that the detrusor muscle relaxes with a β3-agonist (J. Urinol. 161, p680-685 (1999); J. Pharmacol. Exp. Ther. 288, p1367-1373 (1999)). On the other hand, while flavoxate hydrochloride, oxybutynin hydrochloride, propiverine hydrochloride and tolterodine have been used in treatment of patients affected by pollakiuria or urinary incontinence up to now (Folia Pharmacologica Japonica, Vol. 113, p157-166 (1999); Eur. J. Pharmaco. 349, p285-292 (1998)), their side effects include mouth dryness, difficulty in urinating, and constipation (RINSHOU HINYOUKIKA, Vol. 52, p277-285 (1998)), and the situation can not be considered satisfactory.

Further, while sibutramine is used as an antiobestic agent, its side effects have been reported to include elevation of blood pressure, mouth dryness and constipation (Int. J. Obesity, 21, S30-36 (1997)). Sibutramine is a monoamine reuptake inhibitor, and examples of other drugs belonging to this group include milnacipran, duloxetine, and venlafaxine (Medicine and Drug Journal, Vol. 36, No. 2, p151-157 (2000)). In addition, fluoxetine (Am. J. Clin. Nutr. 64, p267-273 (1996)), sertraline (J. Endocrinol. Invest. 19, p727-733 (1996)), paroxetine (Drugs 55, p85-120 (1998)) and fluvoxamine (J. Clin. Psychiatry 57, p346-348 (1996)) are considered useful as antiobestic agents. Further, orlistat has also begun to be used as an antiobestic agent, however, although the number of cases is small, gastrointestinal dysfunction side effects (fatty stool, defecation increase, abdominal pain, and impending defecation desire) have been reported (J. Clin. Pharmacology 37, p453-473 (1997)).

As therapeutic agents for type II diabetes mellitus, insulin, an insulin secretagogue, a biguanide, an α-glucosidase inhibitor, and an insulin sensitizer are used. As compounds having an effect that promotes insulin secretion, glibenclamide, glipizide, gliclazide, glimepiride, tolazamide, tolbutamide, acetohexamide, chlorpropamide, glyclopyramide, and meglitinide are used. Further, compounds called repaglinide and nateglinide have begun to be used, while compounds in clinical testing include mitiglinide (NIPPONRINSHO (an extra issue), Diabetes Mellitus 2, p147-185 (1997)); IGAKU NO AYUMI, Diabetes Mellitus, Vol. 188, p491-495 (1999)). However, these insulin secretagogues have side effects of appetite promotion and hypoglycemia, and the situation is not necessarily a satisfactory one. As biguanide, metformin and buformin are used, and they are known to cause lactic acidosis (IGAKU NO AYUMI, Diabetes Mellitus, Vol. 188, p504-509 (1999)). As α-glucosidase inhibitors, acarbose (Ann. Pharmacother. 30, p1255-1262 (1996) and voglibose (NIPPONRINSHO, Vol. 55 (Suppl.), p114-119 (1997)) are used clinically, and miglitol (Pharmacology, 43, p318-328 (1991)) and emiglitate (Eur. J. Clin. Pharmacol. 41, p561-567 (1991)) are in clinical testing. These α-glucosidase inhibitors have side effects of abdominal distension, flatus increase, loose passage and diarrhea (IGAKU NO AYUMI, Diabetes Mellitus, Vol. 188, p496-499 (1999)). As insulin sensitizer, the thiazolidinedione derivatives troglitazone, pioglitazone and rosiglitazone (Japanese Patent Application Laying-Open (kokai) No. 55-22636; Japanese Patent Application Laying-Open (kokai) No. 60-51189; Japanese Patent Application Laying-Open (kokai) 6-157522; European Patent No. 0306228; Diabetes 37, p1549-1558 (1988); Diabetes 43, p1203-1210 (1994); Diabetes 41, p476-483 (1992); IGAKU NO AYUMI, Diabetes Mellitus, Vol. 188, p500-503 (1999)) are used clinically. Further, compounds in clinical testing include MCC-555 (Br. J. Pharmacol. 125, p767-770 (1998)), GI-262570 (WO97/31907), JTT-501 (Diabetologia 42, p151-159 (1999)), and KRP-297 (Diabetes 47, p1841-1847 (1998)). A side effect of insulin sensitizer is weight gain, and for some insulin sensitizer a side effect of fulminant hepatitis has been cited although the frequency is low (IGAKU NO AYUMI, Diabetes Mellitus, Vol. 188, p500-503 (1999)).

Antilipemic drugs include HMG-CoA reductase inhibitors, anion exchange resins, fibric acid derivatives and nicotinic acid derivatives. As HMG-CoA reductase inhibitors, pravastatin (YAKUGAKU JOURNAL, Vol. 111, p469-487 (1991); U.S. Patent No. 4346227), simvastatin (Atherosclerosis 101, p117-125 (1993); U.S. Patent No. 4444784), fluvastatin (Journal of clinical therapeutics & medicine, Vol. 11 (Suppl. 1), p153-180 (1995)); atorvastatin (Am. J. Cardiol. 79, p1248-1252 (1997)), and cerivastatin (Atherosclerosis, 135, p119-130 (1997)) are used clinically, and nisvastatin (Life Sci. 65, p1493-1502 (1999)) and S-4522 (Bioorg. Med. Chem. 5, p437-444 (1997)) are in clinical testing. Side effects of HMG-CoA reductase inhibitors include gastrointestinal dysfunction, liver enzyme increase, CPK increase, and myopathy (Mol. Med. 31, p544-549 (1994)). Anion exchange resins include cholestyramine (KISO TO RINSHOU (The clinical report), Vol. 16, p150-169 (1982)) and cholesthimide (Journal of clinical therapeutics & medicine, Vol. 12, p1263-1304 (1996)), and their side effects include a bloating sensation, constipation, hard stool and hepatotoxicity. Fibric acid derivatives include clofibrate, simfibrate, clinofibrate, bezafibrate, fenofibrate, ciprofibrate and gemfibrozil, and their side effects include gastrointestinal dysfunction, hepatopathy, gallstone, lowering of libido, CPK increase and myopathy. Nicotinic acid derivatives include nicotinic acid, nicomol, niceritrol and tocopherol nicotinate, and their side effects include a heat sensation, flushing, mouth dryness, lowering of glucose tolerance, gastrointestinal dysfunction and hyperuricemia (Mol. Med. 31, p544-549 (1994)). In addition, combined use of fibric acid derivatives and statins is a contraindication because of hepatotoxicity.

As described above, the situation regarding these drugs is not yet satisfactory. The provision of a novel, useful and superior remedy that can be used in treatment and prevention of urinary incontinence, obesity, diabetes mellitus, hyperlipidemia and the like has been long awaited.

### DISCLOSURE OF THE INVENTION

In order to solve the above problems, the present inventors have confirmed that β3-agonist has an activity that inhibits urinary incontinence. Further, we discovered that when used together with a therapeutic agent for urinary incontinence such as propiverine, oxybutynin hydrochloride or tolterodine, the β3-agonist exerts an enhanced anti-urinary incontinence effect, when used together with an antiobestic agent such as sibutramine or orlistat, it exerts an enhanced antiobestic effect, when used together with an antidiabetic agent such as insulin, glibenclamide, acarbose or rosiglitazone, it exerts an enhanced antidiabetic effect, and when used together with an antilipemic agent such as bezafibrate or pravastatin, it exerts an enhanced antilipemic effect. Thus, we succeeded in completing the present invention.

That is, the present invention provides a therapeutic agent characterized by containing at least one member selected from the group consisting of an anticholinergic agent, a monoamine reuptake inhibitor, a lipase inhibitor, a selective serotonin reuptake inhibitor, insulin, an insulin secretagogue, biguanide, an α-glucosidase inhibitor, an insulin sensitizer, a HMG-CoA reductase inhibitor, an anion exchange resin, a fibric acid derivative and a nicotinic acid derivative and a compound having a β3 agonist activity, or a treatment method characterized by administration of the therapeutic agent.

More specifically, the present invention provides a therapeutic agent for pollakiuria and urinary incontinence characterized by comprising at least an anticholinergic agent and a compound having a β3 agonist activity, as well as a treatment method for pollakiuria and urinary incontinence characterized by administration of the therapeutic agent.

Further, the present invention provides a therapeutic agent for obesity characterized by comprising at least one member selected from the group consisting of a monoamine reuptake inhibitor, a lipase inhibitor and a selective serotonin reuptake inhibitor, and a compound having a β3 agonist activity, as well as a treatment method for obesity characterized by administration of the therapeutic agent.

In addition, the present invention provides a therapeutic agent for diabetes mellitus characterized by comprising at least one member selected from the group consisting of insulin, an insulin secretagogue, biguanide, an α-glucosidase inhibitor and an insulin sensitizer, and a compound having a β3 agonist activity, as well as a treatment method for diabetes mellitus characterized by administration of the therapeutic agent.

Further, the present invention provides a therapeutic agent for hyperlipemia characterized by comprising at least one member selected from the group consisting of a HMG-CoA reductase inhibitor, an anion exchange resin, a fibric acid derivative and a nicotinic acid derivative, and a compound having a β3 agonist activity, as well as a treatment method for hyperlipemia characterized by administration of the therapeutic agent.

This specification includes part or all of the contents as disclosed in the specification of Japanese Patent Application No. 2000-20733, which is a priority document of the present application.

### PREFERRED EMBODIMENT OF THE INVENTION

Preferably, the β3-agonist of the present invention is, for example, a compound shown by any one of the following general formula (I), general formula (II), general formula (III) and a salt thereof.

A compound of the formula (I): 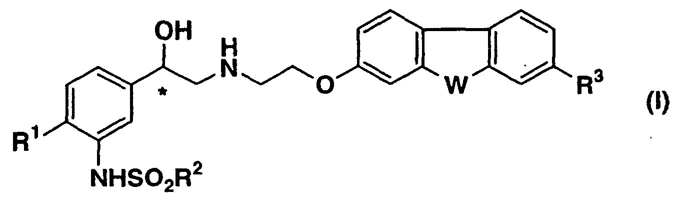 or a salt thereof,
wherein R¹ represents a hydrogen atom, a halogen atom or a hydroxyl group, and R² represents a lower alkyl group or a benzyl group. R³ represents OR, a halogen atom, a trifluoromethyl group, a lower alkyl group, a lower acyl group, NR⁴R^{4'}, a nitro group or a cyano group. Further, R represents a hydrogen atom, a lower alkyl group, a benzyl group or an optionally substituted lower acyl group, and R⁴ and R^{4'} may be the same or different each other and represents a hydrogen atom, a lower alkyl group, a lower acyl group, a benzyl group or SO₂R⁵, R⁵ represents a lower alkyl group or a benzyl group. W represents an oxygen atom, a secondary nitrogen atom (NH) or a sulfur atom.
* represents an asymmetric carbon atom.

A compound of the formula (II): 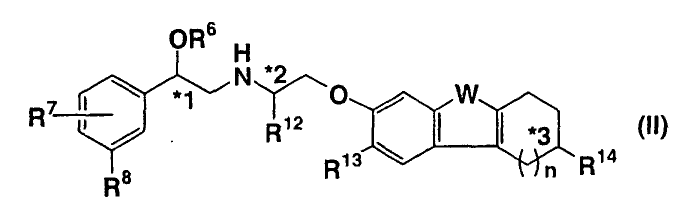 or a salt thereof,
wherein R⁶ represents a hydrogen atom or a methyl group; R⁷ represents a hydrogen atom, a halogen atom, a hydroxyl group, a benzyloxy group, an amino group or a hydroxymethyl group; and R⁸ represents a hydrogen atom, a hydroxymethyl group, NHR⁹, SO₂NR¹⁰R^{10'} or a nitro group. Provided that R⁹ represents a hydrogen atom, a methyl group, SO₂R¹¹, a formyl group or CONHR^{12'}, and R¹¹ represents a lower alkyl group, a benzyl group or NR¹⁰R^{10'}. Further, R¹⁰ and R^{10'} may be the same or different each other and represents a hydrogen atom, a lower alkyl group or a benzyl group, R^{12'} represents a hydrogen atom or a lower alkyl group. Further, R¹² represents a hydrogen atom or a lower alkyl group. n represents 1 or 2, and W represents a secondary nitrogen atom, an oxygen atom or a sulfur atom. When n is 1, one of R¹³ and R¹⁴ represents a hydrogen atom and the other represents a hydrogen atom, an amino group, an acetylamino group or a hydroxyl group. When n represents 2, R¹⁴ represents a hydrogen atom and R¹³ represents a hydrogen atom, an amino group, an acetylamino group or a hydroxyl group.
*1 represents an asymmetric carbon atom, and when neither R¹² nor R¹⁴ is a hydrogen atom.
*2 and *3 mean an asymmetric carbon atom.

A compound of the formula (III): 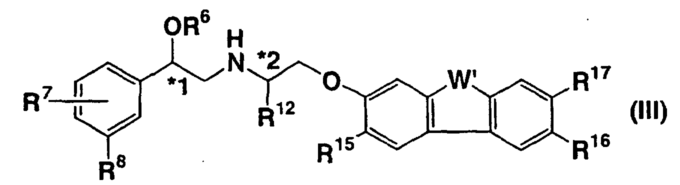 or a salt thereof,
wherein R⁶ represents a hydrogen atom or a methyl group; R⁷ represents a hydrogen atom, a halogen atom, a hydroxyl group, a benzyloxy group, an amino group or a hydroxymethyl group; and R⁸ represents a hydrogen atom, a hydroxymethyl group, NHR⁹, SO₂NR¹⁰R^{10'} or a nitro group. Provided that R⁹ represents a hydrogen atom, a methyl group, SO₂R¹¹, a formyl group or CONHR^{12'}, and R¹¹ represents a lower alkyl group, a benzyl group or NR¹⁰R^{10'}. Further, R¹⁰ and R^{10'} may be the same or different each other and represents a hydrogen atom, a lower alkyl group or a benzyl group, R^{12'} represents a hydrogen atom or a lower alkyl group. Further, R¹² represents a hydrogen atom or a lower alkyl group. W' represents a secondary nitrogen atom, an oxygen atom, a sulfur atom or a methylene group; and when W' is a secondary nitrogen atom, an oxygen atom or a sulfur atom, R¹⁷ represents a hydrogen atom and one of R¹⁵ and R¹⁶ represents a hydrogen atom and the other represents a hydrogen atom, an amino group, an acetylamino group or a hydroxyl group. Further, when W' is a methylene group, R¹⁵ and R¹⁶ each represent a hydrogen atom and R¹⁷ represents a hydrogen atom, an amino group, an acetylamino group or a hydroxyl group.
*1 represents an asymmetric carbon atom, and when R¹² is a lower alkyl group.
*2 means an asymmetric carbon atom,.

The compound of the above general formula (I) and a pharmaceutically acceptable salt thereof are, for example, a novel compound prepared by a method described hereinafter. Further, the compound of general formula (II) and a pharmaceutically acceptable salt thereof are, for example, as described in WO99/01431. Further, the compound shown by general formula (III) and a pharmaceutically acceptable salt thereof are, for example, as described in Japanese Patent Application Laying-Open (kokai) No. 9-249623. In addition, as the β3-agonist of the present invention, any compound having a β3 agonist activity may be used, and examples include compounds described in United States Patent No. 5786356 and WO98/43953.

In general formula (I), "halogen atom" may be fluorine, chlorine, bromine or iodine atom with fluorine, chlorine and bromine atoms being preferred. In addition, "lower alkyl group" means a straight or branched saturated hydrocarbon containing 1 to 4 carbon atoms and includes methyl, ethyl, n-propyl, i-propyl, n-butyl, i-butyl, sec-butyl and tert-butyl groups. Further, "lower acyl group" means a straight or branched acyl group containing 1 to 6 carbon atoms and includes formyl, acetyl, propionyl, butyryl, isobutyryl, valeryl, isovaleryl, pivaloyl and hexanoyl groups.

R¹ is a hydrogen atom, a halogen atom or a hydroxyl group, and preferred examples thereof include hydrogen, fluorine, chlorine and bromine atoms and hydroxyl group.

R² is a lower alkyl group or a benzyl group, and specific examples thereof include methyl, ethyl, benzyl, n-propyl, i-propyl, n-butyl, i-butyl, sec-butyl and tert-butyl groups, with methyl and benzyl groups being particularly preferred.

R³ is OR, a halogen atom, a trifluoromethyl group, a lower alkyl group, a lower acyl group, NR⁴R^{4'}, a nitro group or a cyano group wherein R represents a hydrogen atom, a lower alkyl group, a benzyl group, or an optionally substituted lower acyl group; and R⁴ and R^{4'} may be the same or different each other and represent a hydrogen atom, a lower alkyl group, a lower acyl group, a benzyl group or SO₂R⁵, and R⁵ represents a lower alkyl group or a benzyl group. Preferred examples of R³ include OR and NR⁴R^{4'}. Preferred examples of R include a hydrogen atom, a lower alkyl group and an optionally substituted lower acyl group. More preferred examples of R⁴ and R^{4'} include a hydrogen atom, a lower acyl group and SO₂R⁵.

W represents an oxygen atom, a secondary nitrogen atom (NH), or a sulfur atom, with secondary nitrogen atom being preferred. Substituent of the optionally substituted lower acyl group is not specifically limited so long as it may be a substituent for a lower alkyl commonly included in a commercially available reagent. Preferred examples of such substituent include an amino group optionally substituted with a lower alkyl group, a hydroxyl group, a lower alkoxy group, or the like.

The term "leaving group" mentioned below means a removable group such as chlorine, bromine or iodine atom, or a sulfonic acid ester such as mesyl or tosyl group.

In the general formula (I) set forth above, * is an asymmetric carbon atom, and the compound of the general formula (I) can be in the form of any of two enantiomers, R-enantiomer and S-enantiomer. Not only optically pure isomers, but also mixtures of the two isomers with any mixing ratio are encompassed in the present invention. From the viewpoint of the expression of pharmacological activity, a preferred configuration of the asymmetric carbon * in the ethanolamino chain is the absolute configuration R. With respect to the asymmetric carbon * of N-[3-[2-[2-(7-hydroxy-9H-carbazol-2-yloxy)ethylamino]-1-hydroxyethyl] phenyl]methanesulfonamide, R-hydroxy structure is a particularly preferred example.

In the compounds of the present invention represented by the general formula (I) or salts thereof, compound having a combination of each of the substituents in which R¹ represents a hydrogen atom, a halogen atom or a hydroxyl group; R³ represents OR, a halogen atom, a trifluoromethyl group, a lower alkyl group, a lower acyl group, NR⁴R^{4'}, a nitro group or a cyano group; R represents a hydrogen atom, a lower alkyl group, a benzyl group or an optionally substituted lower acyl group; R⁴ and R^{4'} may be the same or different each other and represent a hydrogen atom, a lower alkyl group, a lower acyl group, a benzyl group or SO₂R⁵; and R⁵ represents a lower alkyl group or a benzyl group, are cited as preferred example.

In the present invention, compounds of the general formula (I) or salts thereof having a combination of each of the substituents, in which R¹ represents a hydrogen atom, a halogen atom or a hydroxyl group; R³ represents OR, a halogen atom, a trifluoromethyl group, a lower alkyl group, NR⁴R^{4'}, a nitro group or a cyano group; R represents a hydrogen atom, a lower alkyl group or a benzyl group; and R⁴ and R^{4'} may be the same or different each other and represent a hydrogen atom, a lower alkyl group or a benzyl group, are also cited as preferred examples.

Compounds of the general formula (I) can be prepared, for example, by the following processes.

### [Preparation Process]

This preparation process is a process comprising reacting a compound of the general formula (IV): 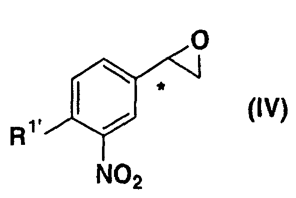 wherein R^{1'} represents a hydrogen atom, a halogen atom, or a protected hydroxyl group, and * represents an asymmetric carbon atom, with a compound of the general formula (V): 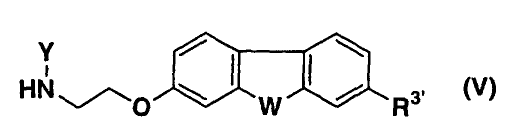 wherein W represents an oxygen atom, a secondary nitrogen atom (NH), or a sulfur atom; Y represents a hydrogen atom or an amine-protecting group; R^{3'} represents OR', a halogen atom, a trifluoromethyl group, a lower alkyl group, a lower acyl group, NR⁴R^{4'}, a nitro group, or a cyano group; R' represents a lower alkyl group, an optionally substituted lower acyl group, a benzyl group, or a hydroxyl-protecting group; R⁴ and R^{4'} may be the same or different each other and represent a hydrogen atom, a lower alkyl group, a lower acyl group, a benzyl group, an amine-protecting group, or SO₂R⁵; and R⁵ represents a lower alkyl group or a benzyl group, to give a compound of the general formula (VI): 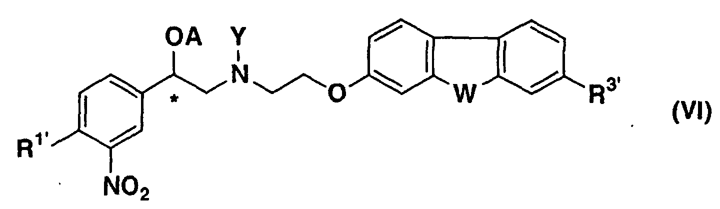 wherein A represents a hydrogen atom, and R^{1'}, R^{3'}, W, Y and * are each as defined above;
converting Y to an amine-protecting group when Y is a hydrogen atom;
reducing the compound of the general formula (VI) in which Y represents an amine-protecting group, to give a compound of the general formula (VII): 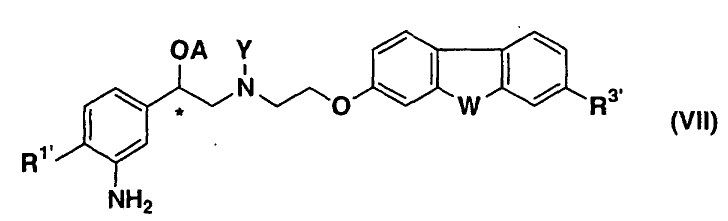 wherein Y represents an amine-protecting group, and A, R^{1'}, R^{3'} W and * are each as defined above;
reacting the compound of the general formula (VII) with a compound of the general formula (VIII):

**XSO**_{**2**}**R**^{**2**} (VIII)

wherein R² represents a lower alkyl group or a benzyl group, and X represents a leaving group wherein the leaving group means a removable group such as chlorine, bromine or iodine atom, or a sulfonic acid ester such as mesyl or tosyl group, in the presence of an alkali, to give a compound of the general formula (IX): 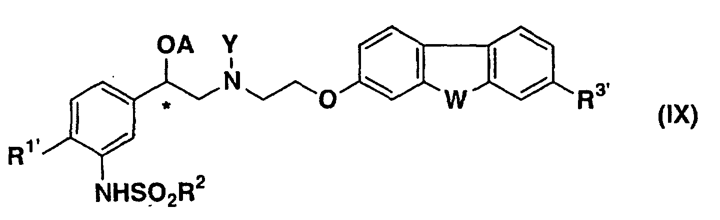 wherein A, R^{1'}, R², R^{3'}, W, Y and * are each as defined above; and
when at least one of R^{1'}, R^{3'} and Y comprises a protecting group, simultaneously or sequentially deprotecting it to give a compound of the general formula (I): 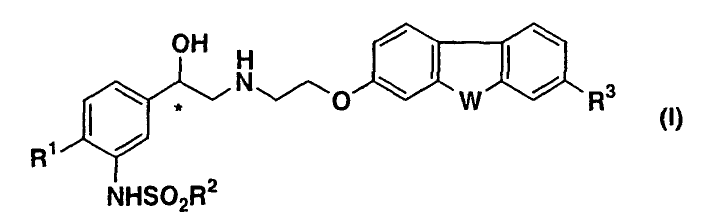 wherein R¹ represents a hydrogen atom, a halogen atom, or a hydroxyl group; R³ represents OR, a halogen atom, a trifluoromethyl group, a lower alkyl group, a lower acyl group, NR⁴R^{4'}, a nitro group, or a cyano group; R represents a hydrogen atom, a lower alkyl group, a benzyl group, or an optionally substituted lower acyl group; R⁴ and R^{4'} may be the same or different each other and represent a hydrogen atom, a lower alkyl group, a lower acyl group, a benzyl group, or SO₂R⁵; and R², R⁵, W and * are each as defined above.

When R^{1'} and/or R^{3'} comprises a hydroxyl-protecting group, the hydroxyl-protecting group is not limited as long as it is commonly used as a hydroxyl-protecting group. Preferred examples of easily and selectively removable hydroxyl-protecting group normally include a trialkylsilyl group, an alkoxyalkyl group , an acyl group and the like. These hydroxyl-protecting groups can be introduced and removed by a known method described in literatures accepted in the art (for example, T. W. Greene, P. G. M. Wuts, et al., Protective Groups in Organic Synthesis, Wiley-Interscience Publication)). For example, a tert-butyldimethylsilyl group (TBDMS) may be introduced into the alcohol by a treatment of the alcohol with a sililating agent such as tert-butyldimethylchlorosilane or tert-butyldimethylsilyl trifluoromethanesulfonate in the presence of an acid scavenger. The amount of the sililating agent to be added is normally about 1.0 to 1.5 mol for 1 mol of the alcohol. Normally, this reaction is preferably carried out in an inert medium. The inert medium may be dichloromethane, tetrahydrofuran, acetonitrile, pyridine and the like. N,N-dimethylformamide is an example of the preferred inert medium. The amount of the inert medium to be used may be about 1 to 5 mL for 1 g of the alcohol. The acid scavenger may be triethylamine, N,N-diisopropylethylamine, pyridine, N,N-dimethylaminopyridine and the like. The acid scavenger may be preferably imidazole. The amount of the acid scavenger to be added may be normally about 1 to 3 mol for 1 mol of the alcohol. Normally, this reaction is preferably carried out at a temperature of from about -20°C to about 80°C, particularly from about 0°C to room temperature, for example, for 1 to 5 hours.

A benzyloxymethyl group (BOM) may be introduced into the alcohol by a treatment of the alcohol with chloromethyl benzyl ether in the presence of an acid scavenger. The amount of chloromethyl benzyl ether to be added may be generally about 1.0 to 1.5 mol for 1 mol of the alcohol. Generally, this reaction is preferably carried out in an inert medium. The inert medium may be tetrahydrofuran, acetonitrile, N,N-dimethylformamide or the like. The inert medium may be preferably dichloromethane. The amount of the inert medium to be used may be about 1 to 5 mL for 1 g of the alcohol. The acid scavenger may be triethylamine, pyridine, N,N-dimethylaminopyridine and the like. An example of the preferred acid scavenger is N,N-diisopropylethylamine. The amount of the acid scavenger to be added may be normally about 1 to 3 mol for 1 mol of the alcohol. Generally, this reaction is preferably carried out at a temperature of from about -20°C to about 80°C, particularly from about 0°C to room temperature, for example, for 1 to 5 hours.

In addition, an acetyl group (Ac) may be introduced into the alcohol , for example, by a treatment of the alcohol with an acetylating agent such as acetic anhydride , acetyl chloride or the like in the presence of an acid scavenger. The amount of the acetylating agent to be added may be generally about 1 to 3 mol for 1 mol of the alcohol. Normally, this reaction is preferably carried out in an inert medium. The examples of the preferred inert medium are tetrahydrofuran, acetonitrile, dichloromethane, pyridine and the like. The amount of the inert medium to be used may be about 1 to 5 mL for 1 g of the alcohol. Examples of the preferred acid scavenger are triethylamine, N,N-diisopropylethylamine, pyridine, N,N-dimethylaminopyridine and the like. The amount of the acid scavenger to be added may be generally about 1 to 3 mol for 1 mol of the alcohol. Generally, this reaction is preferably carried out at a temperature of from about -20°C to about 80°C, particularly from about 0°C to room temperature, for example, for 1 to 5 hours.

In addition, when Y, R⁴ and/or R^{4'} comprises an amino-protecting group, the amino-protecting group may be, for example, an acyl group, an acyloxy group, or an easily removable aralkyl group. Examples of the easily removable aralkyl group include a benzyl group, a substituted benzyl group, a naphthylmethyl group, a substituted naphthylmethyl group and the like. A particularly preferred example is a benzyl group. The aralkyl group to be used may be an aralkyl group having 7 to 16 carbon atoms. Specific examples thereof include benzyl, phenethyl, 3-phenylpropyl, 4-phenylbutyl, (1-naphthyl)methyl, 2-(1-naphthyl)ethyl and 2-(2-naphthyl)ethyl groups. These aralkyl groups may have one or more suitable substituents, such as alkyl group, alkoxy group and halogen atom on suitable positions of the phenyl and naphthyl rings. These protecting groups may be introduced by a known method described in the abovementioned literatures accepted in the art.

A compound of the general formula (VI) is a novel substance and is characteristic as an important intermediate for synthesizing a compound of the general formula (I). A compound of the general formula (VI) is obtained by reacting a compound of the general formula (IV) with a compound of the general formula (V) in a conventional medium, for example in an organic solvent such as dimethylsulfoxide, a linear or cyclic ether, dimethylformamide, dimethylacetamide, or an alcohol solvent, such as 2-butanol. Though a compound of the general formula (IV) and a compound of the general formula (V) are usually used in an equimolar amount, the latter is preferably used in an excess amount. The reaction temperature can be suitably selected and may be generally a temperature of from room temperature to the reflux temperature of the selected solvent. The reaction time can be suitably selected depending on the reaction conditions and the reaction may be normally completed when the yield is the highest. In addition, there is a report (Tetrahedron Letters, 27, p. 2451 (1986)) that the addition of trimethylsilylacetamide (TMSA), N,O-bis(trimethylsilyl)acetamide, hexamethyldisilazane (HMDS) or bis(trimethylsilyl)urea to the reaction can shorten the reaction time and improve the yield. This may be suitably applied to the present reaction.

In addition, a compound of the general formula (VII) is also a novel substance and is characteristic as an important intermediate for synthesizing a compound of the general formula (I). A compound of the general formula (VII) may be obtained by reducing the nitro group of a compound of the general formula (VI) to amine (aniline). When Y of the general formula (VI) is a hydrogen atom, Y is converted to an amine-protecting group prior to such a reduction reaction. The reduction reaction can be carried out by hydrogenating the compound in the presence of platinum oxide as a catalyst in a solvent such as methanol, or by reducing the compound using hydrochloric acid in the presence of iron powder or bivalent tin.

In addition, a compound of the general formula (IX) is also a novel substance and is characteristic as an important intermediate for synthesizing a compound of the general formula (I). A compound of the general formula (IX) may be obtained by sulfonating amine (aniline) of a compound of the general formula (VII) with a compound of the general formula (VIII) which provides various substituents as R², according to the method described in C. Kaiser, et al., J. Med. Chem., 17, p. 49 (1974). When R^{1'}, R^{3'} and/or Y comprise amine-protecting groups and/or hydroxyl-protecting groups, they are removed by the deprotecting method set forth below to give a compound of the general formula (I).

The above sulfonation reaction may be a reaction of a known or commercially available compound of the general formula (VIII) with a compound of the general formula (VII) in a solvent such as pyridine at a temperature of from ice cooling to room temperature. The deprotecting processes may be sequentially or simultaneously carried out. Preferably, the hydroxyl-protecting group in R^{1'} or R^{3'} may be first removed, followed by the removal of the amino-protecting groups in Y and R^{3'}. The deprotecting conditions are as follows. A benzyl group as the hydroxyl-protecting group in R^{1'} and R^{3'} is removed by a hydrogenolysis reaction with a catalyst such as palladium or nickel in a solvent such as methanol. Alternatively, a benzyl or methyl group as the hydroxyl-protecting group in R^{1'} and R^{3'} is removed by a treatment with a Lewis acid such as boron tribromide in a solvent such as methylene chloride. In addition, an acetyl group as the hydroxyl-protecting group in R^{1'} and R^{3'} is removed using known ester hydrolysis reaction conditions. A specific example may be a process comprising heating the compound in the presence of an alkali in an alcohol solvent at a temperature of from room temperature to the reflux temperature of the solvent. In addition, an triethylsilyl group as the hydroxyl-protecting group in R^{1'} and R^{3'} can be removed by treating the compound with acetic acid and 3 to 5 mol of tetrabutylammonium fluoride for 1 mol of the compound in tetrahydrofuran at room temperature for 0.5 to 5 hours. A benzyl group as the amino-protecting group in Y and R^{3'} can be removed by a hydrogenolysis reaction with a catalyst such as palladium or nickel in a solvent such as methanol. In addition, an acetyl group as the amine-protecting group in Y and R^{3'} can be removed by a treatment with hydrochloric acid in a solvent such as methanol at room temperature, or a heating treatment in the presence of an alkali in a solvent such as water or methanol.

A compound of the general formula (IV) is a known substance. A racemic modification thereof can be obtained, for example, by oxidizing a known corresponding styrene compound with an oxidizing agent such as m-chloroperbenzoic acid in a solvent such as dichloromethane at a temperature of from about 0°C to room temperature.

Alternatively, a compound of the general formula (IV) may be obtained by reducing a compound of the general formula (X): 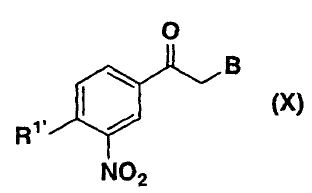 wherein R^{1'} is as defined above, and B represents a chlorine, bromine or iodine atom, according to the below mentioned method or the like to give a compound of the general formula (XI): 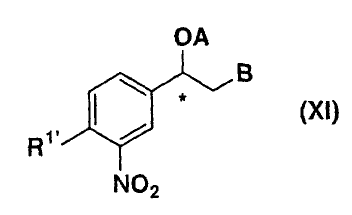 wherein R^{1'} and * are each as defined above, A represents a hydrogen atom, and B represents a chlorine, bromine or iodine atom;
when the compound having an iodine atom as the substituent B is to be obtained, replacing the chlorine or bromine atom with a iodine atom; and then epoxidizing the compound of the formula (XI) by an alkali treatment. That is, when the configuration * of the hydroxyl group of a compound of the general formula (XI) is racemic, a compound of the general formula (X) can be reduced with a reducing agent, such as borane.

In addition, if an optical isomer of either R-form or S-form with respect to * of the general formula (XI) is to be obtained, it can be obtained using a chiral auxiliary agent, such as a material represented by the general formula (XII): 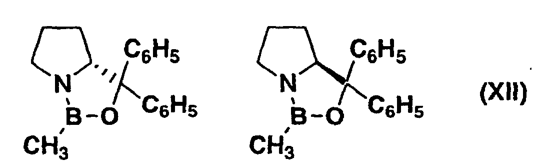 That is, it can be obtained by reducing a compound of the general formula (X) with borane in the presence of the above chiral auxiliary agent. The above reduction reaction is preferably carried out in a solvent, such as tetrahydrofuran. A process for the preparation of these chiral auxiliary agents and reactions thereof may be carried out in accordance with the teachings of E. J. Corey, et al., J. Org. Chem., 56, p. 442 (1991).

When the replacement of the chlorine or bromine atom with an iodine atom is needed after the reduction of a compound of the general formula (X) to a compound of the general formula (XI), there is exemplified a method of heating the reduced compound with an iodinating agent such as 3 to 10 mol of sodium iodide for 1 mol of the brominated form in a solvent such as acetone at the reflux temperature for 1 to 3 hours. Thereafter, the thus obtained compound is epoxidized in the presence of an alkali such as 1 to 2 equivalents of sodium hydroxide aqueous solution in a solvent such as methanol at a temperature of from about 0°C to room temperature to give a compound of the general formula (IV). When a compound of the general formula (IV) is obtained from a compound of the general formula (XI), the configuration with respect to the asymmetric carbon * is retained. That is, R-form generates R-form, and S-form generates S-form.

A compound of the general formula (X), which is a known compound, is commercially available or can be prepared according to the process described in, for example, A. A. Larsen, et al., J. Med. Chem., 10, p. 462 (1967), or C. Kaiser, et al., L Med. Chem., 17, p. 49 (1974).

A compound of the general formula (V) is a novel substance and is characteristic as an important intermediate for the preparation of a compound of the general formula (I).

A compound of the general formula (V) is obtained by reacting a compound of the general formula (XIII): 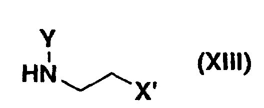 wherein Y represents an amine-protecting group, and X' represents a chlorine atom, a bromine atom or a hydroxyl group, with a compound of the general formula (XIV): 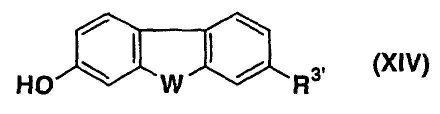 wherein W represents an oxygen atom, a secondary nitrogen atom (NH), or a sulfur atom; R^{3'} represents OR', a halogen atom, a trifluoromethyl group, a lower alkyl group, a lower acyl group, NR⁴R^{4'}, a nitro group, or a cyano group; R' represents a lower alkyl group, an optionally substituted lower acyl group, a benzyl group, or a hydroxyl-protecting group; R⁴ and R^{4'} may be the same or different each other and represent a hydrogen atom, a lower alkyl group, a lower acyl group, a benzyl group, an amine-protecting group, or SO₂R⁵; and R⁵ represents a lower alkyl group or benzyl group. Y, R⁴ or R^{4'} is an amine-protecting group, and is not limited as long as it is commonly used as an amine-protecting group. Examples of the amine-protecting group include a benzyl group, a benzyloxycarbonyl group, a substituted benzyloxycarbonyl group, tert-butoxycarbonyl group, a trifluoroacetyl group and the like, which is normally easily removable.

The reaction of a compound of the general formula (XIII) with a compound of the general formula (XIV) wherein X' represents a chlorine or bromine atom, is carried out , for example, in the presence of a base in an organic solvent at a temperature between room temperature and the reflux temperature of the selected solvent. Such solvents include dimethylformamide, dimethylacetamide, acetonitrile, diglyme and tetrahydrofuran. The base, such as potassium carbonate, sodium carbonate, sodium hydroxide, potassium hydroxide, triethylamine, pyridine, sodium hydride, sodium methoxide or the like is preferably used in an amount of 1 to 10 mol for 1 mol of a compound of the formula (XIV).

When the reaction slowly proceeds, a compound of the general formula (V) in which Y represents an amine-protecting group may be prepared according to the process described in Bull. Chem. Soc. Jpn., 55, p. 2504 (1982) or an improved process thereof. An exemplified process comprises reacting a compound of the general formula (XIV) with 2 to 5 mol of a compound of the general formula (XIII) and 5 to 10 mol of 40% potassium fluoride/alumina for 1 mol of the compound of the general formula (XIV) in dimethylformamide or acetonitrile at a temperature of from room temperature to about 90°C. According to the improved process, 0.1 to 0.5 equivalent of potassium iodide is further added to the mixture.

In addition, the removal of the amine-protecting group Y gives an amine compound represented by the general formula (V) wherein Y represents a hydrogen atom. A benzyl group as the protecting group can be removed by a hydrogenolysis with palladium/carbon as a catalyst in a solvent such as methanol or by a treatment with hydrogen bromide/acetic acid. When the protecting group Y is an acetyl or trifluoroacetyl group, a treatment with an alkali in a solvent such as methanol gives a compound of the general formula (V) wherein Y represents a hydrogen atom.

In addition, a compound of the general formula (XIII) wherein X' is a hydroxyl group can be prepared by a reaction with a compound of the general formula (XIV) according to Mitsunobu reaction. That is, there is exemplified a reaction in the presence of 1 to 10 equivalents of triphenylphosphine and 1 to 10 equivalents of diethyl azodicarboxylate in a solvent such as tetrahydrofuran at a temperature of from about 0°C to room temperature.

A compound of the general formula (XIII) wherein X' is a hydroxyl group can be prepared by protecting amine of a commercially available amino alcohol with an amine-protecting group Y. The hydroxyl group is then brominated or iodinated according to a conventional method to prepare the corresponding brominated form or iodinated form. A compound of the general formula (XI) wherein Y is a benzyl group is preferred since it can be easily obtained by brominating a commercially available benzylaminoethanol. Further, if an aminobrominated form is easily available, it can be protected with an amine-protecting group Y to give a compound of the general formula (XIII). An exemplified process comprises reacting a commercially available 2-bromoethylamine hydrobromate with benzyloxycarbonyl chloride in the presence of triethylamine in methylene chloride with ice cooling.

In addition, a compound of the general formula (V) can be also obtained by the following process. That is, a compound of the general formula (V) can be obtained by reacting a compound of the general formula (XIV) with a compound of the general formula (XV): 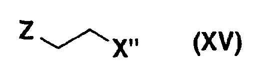 wherein Z represents a leaving group wherein the leaving group means a removable group such as chlorine, bromine or iodine atom, or a sulfonic acid ester such as mesyl or tosyl group, and X" represents a halogen atom, to give a compound of the general formula (XVI): 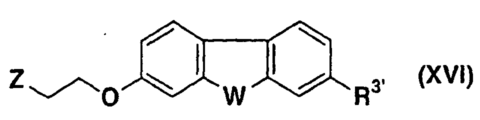 wherein W, Z and R^{3'} are each as defined above; and then replacing Z with a compound of the general formula (XVII):

**YNH**_{**2**} (XVII)

wherein Y represents a hydrogen atom or an amine-protecting group, to give a compound of the general formula (V).

A compound of the general formula (XIV) wherein W is a secondary nitrogen atom and R^{3'} is other than hydroxyl, chloro and methyl is a novel compound and can be prepared by the following process. That is, a compound of the general formula (XIV) wherein R^{3'} is a hydroxyl group can be prepared according to the process described in S. P. Popri, et al., Indian J. Chem. Sect. B, 14B, p. 371 (1976). This compound can be reacted with alkyl halide in the presence of a base such as potassium carbonate to prepare a compound of the general formula (XIV) wherein R^{3'} is OR'. Further, a protecting group can be introduced according to the method for introducing a protecting group set forth above. Further, a compound of the general formula (XIV) wherein R^{3'} is a bromine atom or a cyano group can be prepared by deprotecting a compound described in R. R. Tidwell, et al., Eur. J. Med. Chem., 32, p. 781 (1997) under a conventional condition for deprotecting methyl ether. Further, a compound of the general formula (XIV) wherein R^{3'} is a chlorine atom can be prepared by deprotecting a compound described in S. P. Popri, et al., J. Med. Chem., 16, p. 425 (1976) likewise in a manner as set forth above. Further, a compound of the general formula (XIV) wherein R^{3'} is a lower alkyl group can be prepared by deprotecting as set forth above, a compound prepared according to the process described in R. S. Kapil, et al., Indian J. Chem. Sect. B, 23B, p. 296 (1984). Alternatively, a compound of the general formula (XIV) can be obtained by coupling a compound of the general formula (XVIII): 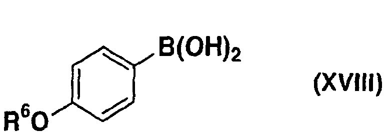 wherein R⁶ represents a hydroxyl-protecting group, with a compound of the general formula (XIX): 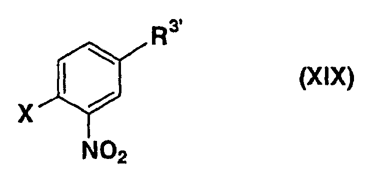 wherein X represents a leaving group, and R^{3'} is as defined above according to Suzuki reaction to give a compound of the general formula (XX): 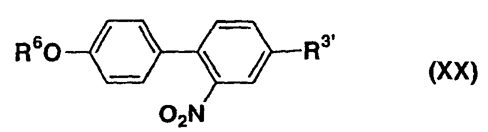 wherein R⁶ and R^{3'} are each as defined above; reductively cyclizing the thus obtained compound of the general formula (XX) to give a compound of the general formula (XXI): 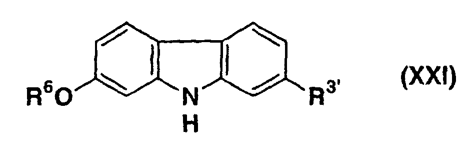 wherein R⁶ and R^{3'} are each as defined above; and then deprotecting the group R⁶.

A compound of the general formula (XVIII) and a compound of the general formula (XIX) are commercially available or can be obtained by adding a protecting group to a commercially available compound. Suzuki reaction may be carried out according to the process described in Miyaura Norio, Suzuki Akira, Yuki Gosei Kagaku Kyoukaishi, 46, p. 848 (1988) or the process described in C. W. Holzapfel, et al., Heterocycles, 48, No.8, pp. 1513-1518 (1998).

A compound of the general formula (XXI) can be prepared according to the process described in J. I. G. Cadogan, et al., J. Chem. Soc., 4831 (1965). That is, a carbazole derivative represented by the general formula (XXI) can be obtained by heating a compound of the general formula (XX) in the presence of trialkyl phosphite or triphenyl phosphite to reductively cyclize the compound. The phosphite to be used is preferably triethyl phosphite. It may be used in an amount of 2 to 10 equivalents, preferably 2 to 4 equivalents. The reaction temperature may be in the range of from about 80°C to about 180°C, preferably from about 130°C to about 170°C. The reaction time may be 1 to 24 hours, preferably 3 to 10 hours. Thereafter, R⁶ may be selectively deprotected according to a conventional method to give a compound of the general formula (XIV).

In addition, a compound of the general formula (XIV) wherein W is an oxygen atom can be obtained by removing the methyl groups of 3,7-dimethoxydibenzofuran described in P. O. Stransky, et al., J. Chem. Soc. Perkin Trans. I, p. 1605 (1982) according to a conventional method and then realkylating or protecting one of the deprotected hydroxyl groups. Further, a compound of the general formula (XIV) wherein W is a sulfur atom can be obtained by reducing 3,7-dihydroxydibenzothiophene 5,5-dioxide described in M. M. Joullie, et al., J. Med. Chem., 21, p. 1084 (1978) with lithium aluminum hydride to give 3,7-dihydroxydibenzothiophene, followed by alkylating or protecting treatment as set forth above.

A further alternative process of Preparation Process may be a process comprising reacting a compound of the general formula (XI): 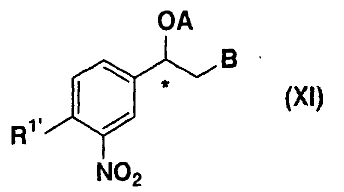 wherein A represents a hydroxyl-protecting group, and R^{1'}, B and * are each as defined above, with a compound of the general formula (V): 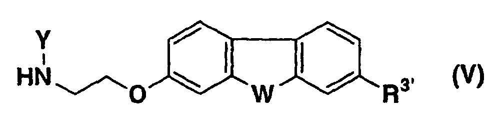 wherein Y represents a hydrogen atom or an amine-protecting group, and W and R^{3'} are each as defined above, to give a compound of the general formula (VI) wherein A represents a hydroxyl-protecting group, and R^{1'}, R^{3'}, W, Y and * are each as defined above; and treating the thus obtained compound according to the method set forth above to give a compound of the general formula (I).

The protecting group A may be introduced and removed according to the method set forth above.

Alternatively, Preparation Process A may be a process comprising reacting a compound of the general formula (X): 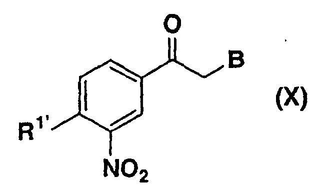 wherein R^{1'} and B are each as defined above, with a compound of the general formula (V) wherein W, Y and R^{3'} are each as defined above, to give a compound of the general formula (XXII): 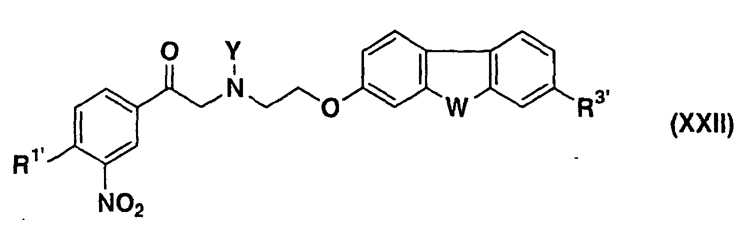 wherein R^{1'}, W, Y and R^{3'} are each as defined above; reducing the carbonyl group according to the method set forth above, to give a compound of the general formula (VI) wherein A represents a hydrogen atom; and treating the thus obtained compound according to the method set forth above, to give a compound of the general formula (I).

The reaction of a compound of the general formula (X) with a compound of the general formula (V) is preferably carried out according to a process which improves the process indicated in A. A. Larsen, et al., J. Med. Chem., 10, p. 462 (1967). That is, the process preferably comprises reacting the said compounds in the absence or presence of an amine as an acid-trapping agent in a polar solvent such as acetonitrile, dimethylformamide, dimethylacetamide or dimethylsulfoxide at a temperature of from ice cooling to about 60°C; successively reducing the carbonyl group with a reducing agent such as sodium borohydride or sodium cyanoborohydride at a temperature of from ice cooling to room temperature; and then removing the protecting groups. An optically active substance can be obtained by optical resolution according to the method set forth below, or by asymmetric reduction with a hydrogen donating compound in the presence of the above-mentioned catalyst or a known asymmetric reduction catalyst disclosed in some literatures such as K. Achiwa, et al., Chem. Pharm. Bull., 43, p. 748 (1995) and R. Noyori, et al., J. Am. Chem. Soc., 118, p. 2521 (1996).

An alternative preparation process may be a process comprising reacting a compound of the general formula (XXIII): 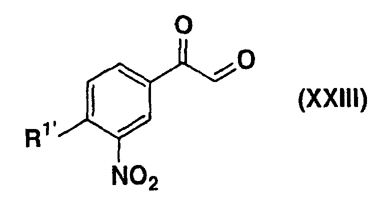 wherein R^{1'} is as defined above, with a compound of the general formula (V) wherein Y represents a hydrogen atom, and W and R^{3'} are each as defined above, followed by reducing the resultant product to give a compound of the general formula (VI) wherein A and Y represent a hydrogen atom, and R^{1'}, R^{3'} and W are each as defined above; and if necessary, and then protecting A and Y by a conventional method, reducing the nitro group likewise by a method set forth above to give a compound of the general formula (VII). The compound of the general formula (I) is obtained likewise by a method described above.

This reaction is usually carried out in a medium in the presence of a suitable reducing agent which can reduce Schiff's base obtained from a condensation reaction and can simultaneously reduce carbonyl group to hydroxyl group. Examples of the reducing agent include sodium borohydride, sodium cyanoborohydride lithium cyanoborohydride and the like. The amount of phenylglyoxal to be used is 1 to 3 mol, preferably 1 to 1.5 mol for 1 mol of the amine. The reaction temperature can be suitably selected and may be generally a temperature of from room temperature to the reflux temperature of the solvent used. The reaction time can be suitably selected depending on the reaction conditions and the reaction may be normally completed when the yield is maximum. An exemplified process is carried out in an alcoholic medium such as methanol or ethanol in the presence of sodium borohydride preferably at a lower temperature. An optically active substance is obtained by optical resolution according to the method set forth below.

A compound of the general formula (XXIII) can be easily obtained by oxidizing acetophenone compounds having the substituent R^{1'} with an oxidizing agent such as selenium dioxide in water or an organic solvent which may be a cyclic ether such as dioxane or tetrahydrofuran. Alternatively, the compound can be prepared according to the process indicated in J. Am. Chem. Soc., 79, p. 6562 (1957).

Further, an alternative preparation process may be a process comprising reacting an amine compound of the general formula (XXIV): 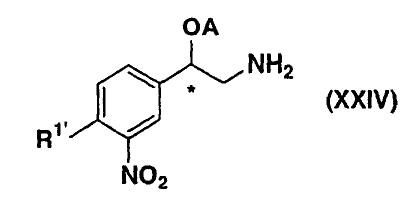 wherein A represents a hydroxyl-protecting group, and R^{1'} and * are each as defined above, with a compound of the general formula (XVI): 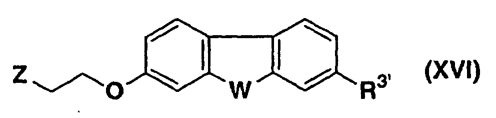 wherein W, R^{3'} and Z are each as defined above, to give a compound of the general formula (VI) wherein Y represents a hydrogen atom, A represents a hydroxyl-protecting group, and R^{1'}, R^{3'} and W are as defined above; and protecting the thus generated amine group, and then preparing a compound of the general formula (I).

The coupling reaction with the amine is carried out in an organic solvent, and if necessary in the presence of a proton acceptor such as a tertiary amine (for example, triethylamine) to give a compound of the general formula (VI) wherein Y represents a hydrogen atom. The leaving group means a group which can be removed in the reaction set forth above, such as chlorine, bromine or iodine atom, or a sulfonic acid ester such as mesyl or tosyl group. The amount of the amine of the general formula (XXIV) to be used as an example of the reaction conditions may be 1 to 10 mol for 1 mol of the compound of the general formula (XVI).

This reaction proceeds slowly and therefore is preferably carried out in an autoclave. Examples of the solvent to be used include alcohols such as methanol, ethanol and butanol, halogenated hydrocarbon such as methylene chloride and chloroform, tetrahydrofuran, dioxan and the like. The reaction temperature is generally in the range of from about 10°C to about 150°C, preferably from about 70°C to about 130°C. The reaction time is generally 5 to 100 hours.

A compound of the general formula (XXIV) may be obtained by hydrogenating a mandelonitrile compound substituted with R^{1'}, for example, in the presence of a catalyst such as Raney nickel. The substituted mandelonitrile may be obtained as a racemic compound by reacting a substituted benzaldehyde with hydrogen cyanide, or with sodium cyanide and sodium hydrogensulfite. The thus obtained racemic compound can be easily resolved into the corresponding optically active isomers by the formation of a salt of diastereomer with a suitably selected optically active acid according to a conventional method and technique. In addition, an optically active compound of the general formula (XXIV) may be obtained by hydrolyzing an optically active substituted mandelonitrile to give an optically active carboxylic acid, and reacting the thus obtained carboxylic acid with ammonia in the presence of a commonly used condensing agent, followed by reducing reaction.

A variety of compounds described herein may be purified, if necessary, and such a purification can be usually carried out by a known chromatography (column, flash column, thin layer, or high-performance liquid chromatography) with referring to, for example, Rf values indicated in the present text of specification.

As mentioned above, a compound of the general formula (I) can exist in the form of either of two optical isomers. Further, the compound of general formula (II) can exist as a maximum of 8 optical isomers, and the compound of general formula (III) can exist as a maximum of 4 optical isomers. The process of the present invention can provide both pure optical isomers and a racemic mixture. The reactions set forth above do not alter the stereochemistry involved in such reactions at all.

Therefore, a racemic modification can be obtained by a process starting from a compound of the general formula (X), or (XXIII) which contains no asymmetric carbon, or from a compound of the general formula (IV), (XI), or (XXIV) as a racemic compound. Likewise, starting from an optically pure isomer of a compound of the general formula (IV), (XI), or (XXIV), for example, R-isomer of the general formula (IV), only R-isomer is obtained. Further, a pure isomer can be obtained using an optically active isomer of a compound of the general formula (IV), (XI), or (XXIV).

When a mixture of two enantiomers (racemic modification) is obtained, it can be optically resolved by a suitable method such as a method comprising fractionally crystallizing the enantiomers as acid addition salts with an optically active acid such as camphorsulfonic acid, mandelic acid or substituted mandelic acid. Such a fractional crystallization may be carried out using a suitable solvent, preferably a lower alkanol, such as ethanol, isopropanol or a mixture thereof.

Each pair of enantiomers can be resolved into pure isomers by formation of diastereomeric salt, chromatography using an optically active column, or other means. When one of starting materials is optically active, the thus obtained mixture of diastereomers can be resolved into pure isomers by the above-mentioned means. Isolation and purification of an optically active isomer makes possible enhanced efficiency and dissolution of side effects due to the use of higher active isomer to give a preferred drug.

A medicine of the present invention is preferably prepared in the form of a pharmaceutical composition by optionally adding a pharmaceutically acceptable carrier to a compound or a salt thereof of the present invention. Examples of pharmaceutically acceptable carriers include excipients, binders such as carboxymethylcellulose, disintegrators, lubricants and auxiliaries.When a compound of the present invention is administered to humans, it can be orally administered in the form of tablet, powder, granule, capsule, sugar-coated tablet, solution, syrup or the like. Further, it can be parenterally administered in the form of injection or the like. The dosage administered will vary dependent on the age and weight of the patient and the extent of disease. The daily dosage for an adult is usually 0.01 to 2000 mg, which is singly administered or is divided into several dosages and then administered. The administration period can vary between several weeks and several months, and the everyday medication is usually applied. However, the daily dosage and administration period can be increased or decreased from the above ranges dependent on the conditions of patient. When concomitantly administering the therapeutic agent of the present invention, each ingredient may be mixed in one container, however, depending on the case, they may be stored in separate containers, and at the time of administration, administered to the same patient at substantially the same time.

"Urinary incontinence" is generally understood as "objectively verifiable involuntary urine leakage, which causes a hindrance in living respects and sanitary respects in daily life", and "pollakiuria" is generally understood as "frequently having a micturition desire, and furthermore, feeling it is not possible to be patient before urination" (New Current 10(18), p2-7 (1999)). Obesity normally indicates a person whose BMI (Body Mass Index) is 25 or more, and it is reported that the ratio of people in this condition who develop hypertension, hyperlipemia or diabetes mellitus is high compared to people whose BMI is under 25 (Nature 404, p635-643 (2000)). Diabetes mellitus is classified into type I diabetes mellitus and type II diabetes mellitus, and in the present invention, type II diabetes mellitus is a more preferable object of treatment. Type II diabetes mellitus is characterized by insulin resistance. The gauge of diabetes mellitus is generally considered to be a fasting blood glucose level of 140 mg/dl or more, and a level of 200 mg/dl or more 2 hours after a 75 g glucose tolerance test (NIPPONRINSHO, Diabetes Mellitus 1, Vol. 55, p247-253 (1997)). Hyperlipemia consists of hypercholesterolemia and hypertriglyceridemia, and hypercholesterolemia, in particular, is a risk factor of arteriosclerosis. The diagnostic criteria of hyperlipemia is described (Molecular Medicine, Vol. 31, p544-550) as a serum cholesterol level of 220 mg/dl or more, a triglyceride level of 150 mg/dl or more, and a HDL (high density lipoprotein)-cholesterol level of 40mg/dl or under.

In the present invention, an anticholinergic agent is an antagonist of a muscarinic receptor, and is as described in RINPI, Vol. 52, No. 5, p277-287 (1998), and specific examples include flavoxate hydrochloride, oxybutynin hydrochloride, propiverine hydrochloride, and tolterodine (Folia Pharmacologica Japonica, Vol. 113, p157-166 (1999); Eur. J. Pharmaco. 349, p285-292 (1998)). In particular, preferred examples are oxybutynin hydrochloride, propiverine hydrochloride, and tolterodine.

It is described in Medicine and Drug Journal, Vol. 36, No. 2, p151-157 (2000) that a monoamine reuptake inhibitor inhibits reuptake of serotonin and noradrenaline and increases monoamine concentration. Specific examples thereof include sibutramine, milnacipran, duloxetine, and venlafaxine, and in particular, sibutramine is a preferred example.

It is described in Medicine and Drug Journal, Vol. 36, No. 2, p137-140 (2000) that a selective serotonin reuptake inhibitor (SSRI) is capable of inhibiting only reuptake of serotonin, and is not capable of reuptake of noradrenaline or dopamine. Specific examples thereof include fluoxetine, sertraline, paroxetine, and fluvoxamine.

Further, it is described in J. Clin. Pharmacology 37, p453-473 (1997) that a lipase inhibitor is an inhibitor of lipase of the stomach or pancreas, and inhibits decomposition of triglyceride in food. In particular, orlistat is a preferred example thereof.

It is described in IGAKU NO AYUMI, Diabetes Mellitus 188, p309-313 (1999) that an insulin secretagogue promotes insulin secretion from β cells by stimulating K_{ATP} channels of pancreatic β-cells. Specific examples thereof include glibenclamide, glipizide, gliclazide, glimepiride, tolazamide, tolbutamide, acetohexamide, chlorpropamide, glyclopyramide, meglitinide, repaglinide, nateglinide and mitiglinide, and in particular, repaglinide, nateglinide and mitiglinide are preferred examples.

Examples of a biguanide include metformin, and buformin.

It is described in IGAKU NO AYUMI, Diabetes Mellitus 188, p496-499 (1999) that an α-glucosidase inhibitor prevents absorption of sugar from the ciliated epithelium by inhibiting α-glucosidase of the small intestine. Specific examples thereof include acarbose, voglibose, miglitol and emiglitate, and in particular, acarbose and voglibose are preferred examples.

An insulin sensitizer is a medicament for ameliorating insulin resistance, which is one of the causes of type II diabetes mellitus, and at the present time a PPAR γ-agonist corresponds to this (Diabetes Mellitus 188, p500-503 (1999)). Specific examples thereof include troglitazone, pioglitazone, rosiglitazone, MCC-555, GI-262570, JTT-501 and KRP-297, and in particular, pioglitazone, rosiglitazone, MCC-555, GI-262570, JTT-501, and KRP-297 are preferred examples.

An HMG-CoA reductase inhibitor is an inhibitor of HMG-CoA reductase, a rate-limiting enzyme of the cholesterol biosynthetic pathway, and it leads to a reduction in blood cholesterol (Mol. Med. 31, p544-549 (1994)). Specific examples thereof include pravastatin, simvastatin, fluvastatin, atorvastatin, cerivastatin, nisvastatin and S-4522.

It is described in KISO TO RINSHOU (The Clinical Report), Vol. 16, p150-169 (1982) that an anion exchange resin binds to bile acid and is excreted in feces, and specific examples thereof include cholestyramine and colestimide.

Fibric acid derivatives have PPAR α agonistic activity and promote fatty acid oxidation (Mol. Med. 37, p83-90 (2000)). Specific examples thereof include clofibrate, simfibrate, clinofibrate, bezafibrate, fenofibrate, ciprofibrate and gemfibrozil.

Specific examples of nicotinic acid derivatives include nicotinic acid, nicomol, niceritrol and tocopherol nicotinate.

The therapeutic agent of the present invention has low toxicity, and therefore a larger amount thereof can be administered. Employing the ratios, as an index, of drug efficacy (for example, effective dose of β3 activity or the like) in each application to various toxicities, comparison of the indexes for use of a β3 agonist by itself and of each drug by itself, and for combined use of a β3 agonist and each drug showed that each index by combined use in treatment of the present invention was high, and the safety and high level of efficacy were readily understood.

Examples of a index of toxicity include mouth dryness, which is a side effect caused by anticholinergic agents. Such mouth dryness can be measured by evaluating suppression of salivation induced with pilocarpine in accordance with the method of Nagao et al. (Folia Pharmacologica Japonica, Vol. 113, p156-166 (1999)). Further, elevation of blood pressure that is a side effect caused by sibutramine can also be employed as an index of toxicity. Such elevation of blood pressure can be determined through use of a pressure transducer. Gastrointestinal dysfunction (fatty stool, defecation increase, abdominal pain, impending defecation desire) that is a side effect caused by orlistat can also be employed as an index of toxicity. Such gastrointestinal dysfunction can be examined by analyzing the stool amount and the stool composition. Promotion of appetite that is a side effect caused by insulin secretagogue can also be employed as an index of toxicity. Such promotion of appetite can be determined by observing body weight fluctuations using an animal, and hypoglycemia can be determined by investigating the concentration of glucose in blood. Loose passage that is a side effect caused by α-glucosidase inhibitor can also be employed as an index of toxicity. Such loose passage and diarrhea can be investigated by, in the case of administration to animal, external observation of stool, or by analysis of stool amount and stool composition. Body weight gain that is a side effect caused by insulin sensitizer can also be employed as an index of toxicity. Such body weight gain can be investigated by measurement of body weight after administration to animal. Gastrointestinal dysfunction that is a side effect caused by HMG-CoA reductase inhibitor can also be employed as an index of toxicity. Such gastrointestinal dysfunction can be investigated by analysis of stool amount and stool composition. Gastrointestinal dysfunction that is a side effect caused by fibric acid derivatives can also be employed as an index of toxicity. Such gastrointestinal dysfunction can be investigated by analysis of stool amount and stool composition.

### EXAMPLES

The present invention is further described in the following examples, however, the examples are not intended to limit the scope of the present invention. Compound a, which is a β3 agonist, used in these examples is (R)-N-[3-[2-[2-(7-hydroxy-9H-carbazol-2-yloxy)ethylamino]-1-hydroxyethyl]phenyl]methanesulfonamide.
Hereinafter, similarly,
compound b is (R)-N-[5-[2-[2-(5,6,7,8-tetrahydro-9H-carbazol-2-yloxy)ethylamino]-1-hydroxyethyl]-2-hydroxyphenyl]methanesulfonamide,
compound c is (R)-N-[5-[2-[2-(dibenzothiophene-3-yloxy)ethylamino]-1-hydroxyethyl]-2-hydroxyphenyl]methanesulfonamide,
compound d is (R)-N-[5-[2-[2-(9H-carbazol-2-yloxy)ethylamino]-1-hydroxyethyl]-2-hydroxyphenyl]methanesulfonamide,
compound e is (R)-N-[3-[2-[2-(9H-carbazol-2-yloxy)ethylamino]-1-hydroxyethyl] phenyl]methanesulfonamide,
compound f is (R)-N'-[5-[2-[2-(9H-carbazol-2-yloxy) ethylamino]-1-hydroxyethyl]-2-hydroxyphenyl]-N,N-dimethylsulfamide,
compound g is (R)-N'-[5-[2-[2-(dibenzofuran-3-yloxy)ethylamino]-1-hydroxyethyl]-2-hydroxyphenyl]-N,N-dimethylsulfamide,
compound h is (R)-N-[5-[2-[2-(9H-carbazol-2-yloxy)ethylamino]-1-hydroxyethyl]-2-chlorophenyl]methanesulfonamide, and
compound i is (R)-N-[5-[2-[2-(9H-carbazol-2-yloxy)ethylamino]-1-hydroxyethyl]-2-bromophenyl]methanesulfonamide.

### (Synthesis of compound a)

In accordance with a method described in the above-described production method, the compound a can be obtained by reacting a compound of general formula (XIV) wherein R^{3'} is a benzyloxy group and W is a secondary nitrogen atom with dibromoethane compound represented by general formula (XV), and further reacting with benzylamine to thereby obtain N-benzyl-2-(7-benzyloxy-9H-carbazol-2-yloxy)ethylamine represented by general formula (V), and then further reacting this compound with a compound of general formula (IV) wherein R^{1'} is a hydrogen atom and reducing the nitro group subsequently, then conducting mesylation reaction followed by deprotecting the benzyl protecting group to give the compound a. A hydrochlorides thereof can be obtained according to a conventional method.
Rf = 0.8 (chloroform/methanol = 4/1 (free form));
Mass (m/e) 456 (MH⁺)

Further, compound b is disclosed in the specification of WO99/01431, and compounds c to i are disclosed in the specification of Japanese Patent Application Laying-Open (kokai) No. 9-249623.

When examining the pharmacological effects for the above-described compounds a to i, hydrochlorides of these compounds are used. Such hydrochlorides can be prepared by a conventional method.

### (EXAMPLE 1) Effect for pllakiuria or urinary incontinence

Measurement of the contractive force of human urinary bladder detrusor was conducted in accordance with the method of Takeda, M. et al. (J. Pharm. Exp. Ther. 288, p1367-1373 (1999). Specifically, contraction was provoked by carbachol (0.5 x 10⁻⁶ M), and a relaxation effect of compounds a, b, c, d, e, f, g, h, i, and propiverine was investigated. Compounds a, b, c, d, e, f, g, h, i, propiverine, oxybutynin, and tolterodine significantly relaxed the bladder detrusor at 10⁻⁷, 10⁻⁷, 10⁻⁸, 10⁻⁸, 10⁻⁶, 10⁻⁷, 10⁻⁷, 10⁻⁶, 10⁻⁶, 10⁻⁵, 10⁻⁸ and 10⁻⁸ M, respectively, however at 10⁻⁹, 10⁻⁹, 10⁻¹⁰, 10⁻¹⁰, 10⁻⁸, 10⁻⁹, 10⁻⁹, 10⁻⁸, 10⁻⁸, 10⁻⁶, 10⁻⁹ and 10⁻⁹ M, respectively, they did not relax the muscle significantly. When a combination of 10⁻⁶ M of propiverine with each of 10⁻⁹ M of compound a, 10⁻⁹ M of compound b, 10⁻¹⁰ M of compound c, 10⁻¹⁰ M of compound d, 10⁻⁸ M of compound e, 10⁻⁹ M of compound f, 10⁻⁹ M of compound g, 10⁻⁸ M of compound h, or 10⁻⁸ M compound i, respectively, were administered to the bladder detrusor, the muscle was relaxed significantly in comparison with administration of each compound alone. Further, when a combination of 10⁻⁹ M of oxybutynin with each of 10⁻⁹ M of compound a, 10⁻⁹ M of compound b, 10⁻¹⁰ M of compound c, 10⁻¹⁰ M of compound d, 10⁻⁸ M of compound e, 10⁻⁹ M of compound f, 10⁻⁹ M of compound g, 10⁻⁸ M of compound h or 10⁻⁸ M of compound i, respectively, were administered to the bladder detrusor, the muscle was relaxed significantly in comparison with administration of each compound alone. Further, when a combination of 10⁻⁹ M of tolterodine with each of 10⁻⁹ M of compound a, 10⁻⁹ M of compound b, 10⁻¹⁰ M of compound c, 10⁻¹⁰ M of compound d, 10⁻⁸ M of compound e, 10⁻⁹ M of compound f, 10⁻⁹ M of compound g, 10⁻⁸ M of compound h or 10 ⁻⁸M of compound i, respectively, were administered to the bladder detrusor, the muscle was relaxed significantly in comparison with administration of each compound alone.

Therefore, the therapeutic agent used in combination of the present invention exhibited a strong synergistic effect in therapeutic effects for pollakiuria and urinary incontinence, and it was confirmed that it is useful as a therapeutic agent for pollakiuria and urinary incontinence .

Regarding mouth dryness as a side effect often observed in administration of propiverine, oxybutynin, or tolterodine, measurement was conducted in accordance with the method of Nagao et al., (Folia Pharmacologica Japonica, Vol. 113, p156-166 (1999)), and suppression of salivation in the condition of a reduced dosage was observed.

### (EXAMPLE 2) Antiobestic effect (I)

Mice induced with obesity by feeding with a high fat diet for 2 months were orally administered with sibutramine (SIB: 1 mg/kg), a β3 agonist (compound c: 0.3mg/kg; compound d: 0.3mg/kg; compound e: 3mg/kg; compound g: 3mg/kg; compound h: 1mg/kg; compound i: 1mg/kg), or SIB and a β3 agonist (compound c, compound d, compound e, compound g, compound h, and compound i) in combination (each dosage was the same as the aforementioned) for 2 weeks and bred, and body weight was measured. For the groups administered solely with SIB, compound c, compound d, compound e, compound g, compound h, or compound i, respectively, almost no change in body weight was observed. In contrast, for the groups administered with a combination of SIB and compound c, SIB and compound d, SIB and compound e, SIB and compound g, SIB and compound h, or SIB and compound i, respectively, a significant weight reduction was observed.

Therefore, it was confirmed that the therapeutic agent of the present case possesses a superior antiobestic effect.

Regarding elevation of blood pressure as a side effect of sibutramine, after administration of the drug, it was determined by use of a pressure transducer that elevation of blood pressure was alleviated to a greater extent.

### (EXAMPLE 3) Antiobestic effect (II)

Mice induced with obesity by feeding with a high fat diet for 2 months were orally administered with orlistat (1mg/kg), a β3 agonist (compound c: 0.3mg/kg; compound d: 0.3mg/kg; compound e: 3mg/kg; compound g: 3mg/kg; compound h: 1mg/kg; compound i: 1mg/kg), or orlistat and a β3 agonist (compound c, compound d, compound e, compound g, compound h, and compound i) in combination (each dosage was the same as the aforementioned) for 2 weeks and bred, and body weight was measured. For the groups administered solely with orlistat, compound c, compound d, compound e, compound g, compound h, or compound i, respectively, almost no change in body weight was observed. In contrast, for the groups administered with a combination of orlistat and compound c, orlistat and compound d, orlistat and compound e, orlistat and compound g, orlistat and compound h, or orlistat and compound i, respectively, a weight reduction was observed.

Therefore, it was confirmed that the therapeutic agent of the present case possesses a superior antiobestic effect

Gastrointestinal dysfunction (fatty stool, defecation increase, abdominal pain, impending defecation desire) as a side effect of orlistat, can be investigated, after administration of the drug, by analyzing stool amount and stool composition.

### (EXAMPLE 4) Antidiabetic effect (I)

Mice were administered with insulin (INS: 0.2 U/kg, intraperitoneal injection), a β3 agonist (oral administration of compound a: 10 mg/kg; compound c: 0.3mg/kg; compound d: 0.3mg/kg; compound e: 3 mg/kg; compound g: 3mg/kg; compound h: 1mg/kg; compound i: 1mg/kg, respectively), or INS and a β3 agonist (compound a, compound c, compound d, compound e, compound g, compound h, and compound i) in combination (each dosage and administration route was the same as the aforementioned), blood was collected after 30 min, and the amount of glucose contained in blood plasma was measured. The results showed that for the groups administered only with INS, compound a, compound c, compound d, compound e, compound g, compound h, or compound i, respectively, no change in blood glucose level was observed. In contrast, for the groups administered with a combination of INS and compound a, INS and compound c, INS and compound d, INS and compound e, INS and compound g, INS and compound h, or INS and compound i, respectively, the blood glucose level decreased significantly.

Therefore, it was confirmed that the therapeutic agent of the present case possesses a superior antidiabetic effect.

### (EXAMPLE 5) Antidiabetic effect (II)

Mice fasted overnight were orally administered with glibenclamide (GLI : 10mg/kg), a β3 agonist (compound a: 10 mg/kg; compound c: 0.3mg/kg; compound d: 0.3mg/kg; compound e: 3 mg/kg; compound g: 3mg/kg; compound h: 1mg/kg; compound i: 1mg/kg), or GLI and a β3 agonist (compound a, compound c, compound d, compound e, compound g, compound h, and compound i) in combination (each dosage was the same as the aforementioned), and immediately glucose (1 g/kg, subcutaneous injection) was loaded. Blood was collected after 1 hour, and the amount of glucose contained in blood plasma was measured. In the groups administered solely with GLI, compound a, compound c, compound d, compound e, compound g, compound h, or compound i, there was almost no difference in the blood glucose level compared to mice not administered with a compound and for which only glucose loading was carried out. In contrast, in the groups administered with a combination of GLI and compound a, GLI and compound c, GLI and compound d, GLI and compound e, GLI and compound g, GLI and compound h, or GLI and compound i, respectively, the blood glucose level decreased significantly.

Therefore, it was confirmed that the therapeutic agent of the present case possesses a superior antidiabetic effect.

In this combined use, hypoglycemia, which is a side effect of glibenclamide, was not observed.

### (EXAMPLE 6) Antidiabetic effect (III)

Genetically diabetic mice were orally administered with rosiglitazone (ROS: 1 mg/kg), a β3 agonist (compound c: 0.3mg/kg; compound d: 0.3mg/kg; compound e: 3 mg/kg; compound g: 3mg/kg; compound h: 1mg/kg; compound i: 1mg/kg), or ROS and a β3 agonist (compound c, compound d, compound e, compound g, compound h, and compound i) in combination (each dosage was the same as the aforementioned) for 2 weeks. Blood was collected thereafter, and the amount of glucose contained in blood plasma was measured. For the groups administered solely with ROS, compound c, compound d, compound e, compound g, compound h, or compound i, no difference in the blood glucose level was observed. In contrast, in the groups administered with a combination of ROS and compound c, ROS and compound d, ROS and compound e, ROS and compound g, ROS and compound h, or ROS and compound i, respectively, the blood glucose level decreased significantly.

Therefore, it was confirmed that the therapeutic agent of the present case possesses a superior antidiabetic effect.

Further, in comparison with the group administered only ROS, weight reduction was observed in the groups administered with ROS and a compound in combination, and thus it was found that weight gain that is a side effect of ROS administration disappeared.

### (EXAMPLE 7) Antidiabetic effect (IV)

Genetically diabetic mice were orally administered with acarbose (ACA: 1 mg/kg), a β3 agonist (compound c: 0.3mg/kg; compound d: 0.3mg/kg; compound e: 3 mg/kg; compound g: 3mg/kg; compound h: 1mg/kg; compound i: 1mg/kg), or ACA and a β3 agonist (compound c, compound d, compound e, compound g, compound h, and compound i) in combination (each dosage was the same as the aforementioned) for 2 weeks. Blood was collected thereafter, and the amount of glucose contained in blood plasma was measured. For the groups administered solely with ACA, compound c, compound d, compound e, compound g, compound h, or compound i, respectively, no difference was observed in the blood glucose level. In contrast, in the groups administered with a combination of ACA and compound c, ACA and compound d, ACA and compound e, ACA and compound g, ACA and compound h, or ACA and compound i, respectively, the blood glucose level decreased.

Accordingly, it was confirmed that the therapeutic agent of this case possesses an excellent antidiabetic effect.

### (EXAMPLE 8) Antilipemic effect (I)

By testing using a vehicle dog, it is possible to observe enhancement of antilipemic effect by combined use of pravastatin (PRA) and a β3 agonist. For example, according to the method of Tsujita, T. et al. (Biochim. Biophys. Acta, 877, p50-60 (1986)), it is shown that by administration to a vehicle dog of PRA: 0.625-1.25 mg/kg, the blood cholesterol level and level of triglyceride in blood decrease. Further, since it is shown that a β3 agonist acts in a vehicle dog (KACHIKU SEIKAGAKU, Vol. 33, p13-18 (1996)), it is expected that compound a, b, c, d, e, f, g, h, and i also act therein. By combined use of pravastatin and a β3 agonist, which have different action mechanisms to each other, a synergistic decrease in the blood cholesterol level and level of triglyceride in blood can be acknowledged.

Accordingly, it will be confirmed that the therapeutic agent of this case possesses an excellent antilipemic effect.

### (EXAMPLE 9) Antilipemic effect (II)

Mice were orally administered with bezafibrate (BEZ: 3mg/kg), a β3 agonist (compound c: 0.3mg/kg; compound d: 0.3mg/kg; compound e: 3mg/kg; compound g: 3mg/kg; compound h: 1mg/kg; compound i: 1mg/kg), or BEZ and a β3 agonist (compound c, compound d, compound e, compound g, compound h, and compound i) in combination (each dosage was the same as the aforementioned) for 2 weeks. Blood was collected thereafter, and the amount of triglyceride contained in blood plasma was measured. For the groups administered solely with BEZ, compound c, compound d, compound e, compound g, compound h, or compound i, respectively, no difference was observed in the blood glucose level. In contrast, for the groups administered with a combination of BEZ and compound c, BEZ and compound d, BEZ and compound e, BEZ and compound g, BEZ and compound h, or BEZ and compound i, respectively, triglyceride decreased.

Accordingly, it is expected that the therapeutic agent of this case possesses an excellent antilipemic effect.

All publications, patents and patent applications cited herein are incorporated herein by reference in their entirety.

### INDUSTRIAL APPLICABILITY

The present invention provides a therapeutic agent comprising at least a compound having a β3 agonist activity and one member selected from the group consisting of an anticholinergic agent, a monoamine reuptake inhibitor, a lipase inhibitor, a selective serotonin reuptake inhibitor, insulin, an insulin secretagogue, biguanide, an α-glucosidase inhibitor, an insulin sensitizer, a HMG-CoA reductase inhibitor, an anion exchange resin, a fibric acid derivative and a nicotinic acid derivative. The therapeutic agent of the present invention has a urinary incontinence treatment effect, an antiobestic effect, an antidiabetic effect, and an antilipemic effect.

## Claims

1. A therapeutic agent comprising at least a compound having a β3 agonist activity and one member selected from the group consisting of an anticholinergic agent, a monoamine reuptake inhibitor, a lipase inhibitor, a selective serotonin reuptake inhibitor, insulin, an insulin secretagogue, biguanide, an α-glucosidase inhibitor, an insulin sensitizer, a HMG-CoA reductase inhibitor, an anion exchange resin, a fibric acid derivative and a nicotinic acid derivative.

2. The therapeutic agent of claim 1, wherein the compound having a β3 agonist activity is a compound represented by any one of the following general formula (I), general formula (II), general formula (III) and a salt thereof:
A compound of the formula (I): 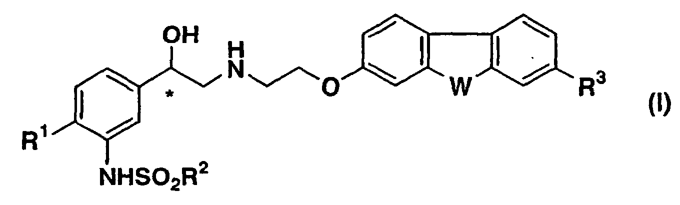 or a salt thereof,
wherein R¹ represents a hydrogen atom, a halogen atom or a hydroxyl group, and R² represents a lower alkyl group or a benzyl group. R³ represents OR, a halogen atom, a trifluoromethyl group, a lower alkyl group, a lower acyl group, NR⁴R^{4'}, a nitro group or a cyano group. Further, R represents a hydrogen atom, a lower alkyl group, a benzyl group or an optionally substituted lower acyl group, and R⁴ and R^{4'} may be the same or different each other and represents a hydrogen atom, a lower alkyl group, a lower acyl group, a benzyl group or SO₂R⁵. R⁵ represents a lower alkyl group or a benzyl group. W represents an oxygen atom, a secondary nitrogen atom (NH) or a sulfur atom,
* means an asymmetric carbon atom,
A compound of the formula (II): 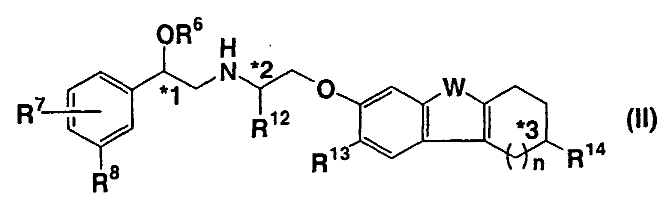 or a salt thereof,
wherein R⁶ represents a hydrogen atom or a methyl group; R⁷ represents a hydrogen atom, a halogen atom, a hydroxyl group, a benzyloxy group, an amino group or a hydroxymethyl group; and R⁸ represents a hydrogen atom, a hydroxymethyl group, NHR⁹, SO₂NR¹⁰R^{10'} or a nitro group. Provided that R⁹ represents a hydrogen atom, a methyl group, SO₂R¹¹, a formyl group or CONHR^{12'}, and R¹¹ represents a lower alkyl group, a benzyl group or NR¹⁰R^{10'}. Further, R¹⁰ and R^{10'} may be the same or different each other and represents a hydrogen atom, a lower alkyl group or a benzyl group. R^{12'} represents a hydrogen atom or a lower alkyl group. Further, R¹² represents a hydrogen atom or a lower alkyl group. n represents 1 or 2, and W represents a secondary nitrogen atom, an oxygen atom or a sulfur atom. When n is 1, one of R¹³ and R¹⁴ represents a hydrogen atom and the other represents a hydrogen atom, an amino group, an acetylamino group or a hydroxyl group. When n represents 2, R¹⁴ represents a hydrogen atom and R¹³ represents a hydrogen atom, an amino group, an acetylamino group or a hydroxyl group.
*1 represents an asymmetric carbon atom, and when neither R¹² nor R¹⁴ is a hydrogen atom.
*2 and *3 mean an asymmetric carbon atom,
A compound of the formula (III): 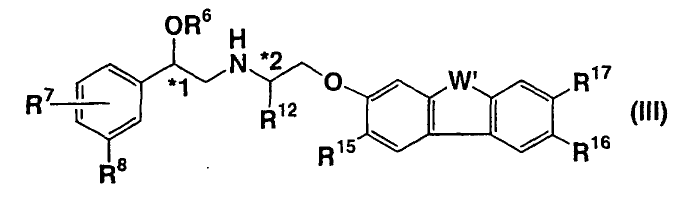
or a salt thereof,
wherein R⁶ represents a hydrogen atom or a methyl group; R⁷ represents a hydrogen atom, a halogen atom, a hydroxyl group, a benzyloxy group, an amino group or a hydroxymethyl group; and R⁸ represents a hydrogen atom, a hydroxymethyl group, NHR⁹, SO₂NR¹⁰R^{10'} or a nitro group. Provided that R⁹ represents a hydrogen atom, a methyl group, SO₂R¹¹, a formyl group or CONHR^{12'}, and R¹¹ represents a lower alkyl group, a benzyl group or NR¹⁰R^{10'}. Further, R¹⁰ and R^{10'} may be the same or different each other and represents a hydrogen atom, a lower alkyl group or a benzyl group. R^{12'} represents a hydrogen atom or a lower alkyl group. Further, R¹² represents a hydrogen atom or a lower alkyl group. W' represents a secondary nitrogen atom, an oxygen atom, a sulfur atom or a methylene group; and when W' is a secondary nitrogen atom, an oxygen atom or a sulfur atom, R¹⁷ represents a hydrogen atom and one of R¹⁵ and R¹⁶ represents a hydrogen atom and the other represents a hydrogen atom, an amino group, an acetylamino group or a hydroxyl group. Further, when W' is a methylene group, R¹⁵ and R¹⁶ each represent a hydrogen atom and R¹⁷ represents a hydrogen atom, an amino group, an acetylamino group or a hydroxyl group.
*1 represents an asymmetric carbon atom, and when R¹² is a lower alkyl group.
*2 means an asymmetric carbon atom.

3. The therapeutic agent of claim 1 or 2, which is a therapeutic agent for pollakiuria and urinary incontinence, comprising at least an anticholinergic agent and a compound having a β3 agonist activity.

4. The therapeutic agent of claim 3, wherein the anticholinergic agent is any one of flavoxate hydrochloride, oxybutynin hydrochloride, propiverine hydrochloride and tolterodine.

5. The therapeutic agent of claim 3, wherein the anticholinergic agent is any one of oxybutynin hydrochloride, propiverine hydrochloride and tolterodine.

6. The therapeutic agent of claim 1 or 2, which is a therapeutic agent for obesity, comprising at least one member selected from the group consisting of a monoamine reuptake inhibitor, a lipase inhibitor and a selective serotonin reuptake inhibitor, and a compound having a β3 agonist activity.

7. The therapeutic agent of claim 6, comprising at least a monoamine reuptake inhibitor and a compound having a β3 agonist activity.

8. The therapeutic agent of claim 6 or 7, wherein the monoamine reuptake inhibitor is any one of sibutramine, milnacipran, duloxetine and venlafaxine.

9. The therapeutic agent of claim 6 or 7, wherein monoamine reuptake inhibitor is sibutramine.

10. The therapeutic agent of claim 6, comprising at least a lipase inhibitor and a compound having a β3 agonist activity.

11. The therapeutic agent of claim 6 or 10, wherein the lipase inhibitor is orlistat.

12. The therapeutic agent of claim 6, comprising at least a selective serotonin reuptake inhibitor and a compound having a β3 agonist activity.

13. The therapeutic agent of claim 6 or 12, wherein the selective serotonin reuptake inhibitor is fluoxetine, sertraline, paroxetine or fluvoxamine.

14. The therapeutic agent of claim 1 or 2, which is a therapeutic agent for type II diabetes mellitus, comprising at least one member selected from the group consisting of insulin, an insulin secretagogue, biguanide, an α-glucosidase inhibitor and an insulin sensitizer, and a compound having a β3 agonist activity.

15. The therapeutic agent of claim 14, comprising at least insulin and a compound having a β3 agonist activity.

16. The therapeutic agent of claim 14, comprising at least an insulin secretagogue and a compound having a β3 agonist activity.

17. The therapeutic agent of claim 14 or 16, wherein the insulin secretagogue is glibenclamide, glipizide, gliclazide, glimepiride, tolazamide, tolbutamide, acetohexamide, chlorpropamide, glyclopyramide, meglitinide, repaglinide, nateglinide or mitiglinide.

18. The therapeutic agent of claim 14 or 16, wherein the insulin secretagogue is glibenclamide.

19. The therapeutic agent of claim 14 or 16, wherein the insulin secretagogue is repaglinide, nateglinide or mitiglinide.

20. The therapeutic agent of claim 14, comprising at least biguanide and a compound having a β3 agonist activity.

21. The therapeutic agent of claim 14 or 20, wherein the biguanide is metformin or buformin.

22. The therapeutic agent of claim 14, comprising at least an α-glucosidase inhibitor and a compound having a β 3 agonist activity.

23. The therapeutic agent of claim 14 or 22, wherein the α-glucosidase inhibitor is acarbose, voglibose, miglitol or emiglitate.

24. The therapeutic agent of claim 14 or 22, wherein the α-glucosidase inhibitor is acarbose.

25. The therapeutic agent of claim 14, comprising at least an insulin sensitizer and a compound having a β3 agonist activity.

26. The therapeutic agent of claim 14 or 25, wherein the insulin sensitizer is troglitazone, pioglitazone, rosiglitazone, MCC-555, GI-262570, JTT-501 or KRP-297.

27. The therapeutic agent of claim 14 or 25, wherein the insulin sensitizer is rosiglitazone.

28. The therapeutic agent of claim 14 or 25, wherein the insulin sensitizer is MCC-555, GI-262570, JTT-501 or KRP-297.

29. The therapeutic agent of claim 1 or 2, which is a therapeutic agent for hyperlipemia, comprising at least one member selected from the group consisting of a HMG-CoA reductase inhibitor, an anion exchange resin, a fibric acid derivative and a nicotinic acid derivative, and a compound having a β3 agonist activity.

30. The therapeutic agent of claim 29, comprising at least a HMG-CoA reductase inhibitor and a compound having a β3 agonist activity.

31. The therapeutic agent of claim 29 or 30, wherein the HMG-CoA reductase inhibitor is pravastatin, simvastatin, fluvastatin, atorvastatin, cerivastatin, nisvastatin or S-4522.

32. The therapeutic agent of claim 29 or 30, wherein the HMG-CoA reductase inhibitor is pravastatin.

33. The therapeutic agent of claim 29 or 30, wherein the HMG-CoA reductase inhibitor is simvastatin, fluvastatin, atorvastatin, cerivastatin, nisvastatin or S-4522.

34. The therapeutic agent of claim 29, comprising at least an anion exchange resin and a compound having a β3 agonist activity.

35. The therapeutic agent of claim 29 or 34, wherein the anion exchange resin is cholestyramine or colestimide.

36. The therapeutic agent of claim 29, comprising at least a fibric acid derivative and a compound having a β3 agonist activity.

37. The therapeutic agent of claim 29 or 36, wherein the fibric acid derivative is clofibrate, simfibrate, clinofibrate, bezafibrate, fenofibrate, ciprofibrate or gemfibrozil.

38. The therapeutic agent of claim 29 or 36, wherein the fibric acid derivative is bezafibrate.

39. The therapeutic agent of claim 29, comprising at least a nicotinic acid derivative and a compound having a β3 agonist activity.

40. The therapeutic agent of claim 29 or 39, wherein the nicotinic acid derivative is nicotinic acid, nicomol, niceritrol or tocopherol nicotinate.

41. A treatment method which comprises administration of a therapeutic agent comprising at least a compound having a β3 agonist activity and one member selected from the group consisting of an anticholinergic agent, a monoamine reuptake inhibitor, a lipase inhibitor, a selective serotonin reuptake inhibitor, insulin, an insulin secretagogue, biguanide, an α-glucosidase inhibitor, an insulin sensitizer a HMG-CoA reductase inhibitor, an anion exchange resin, a fibric acid derivative and a nicotinic acid derivative.

42. The treatment method of claim 41, wherein the compound having a β3 agonist activity is a compound represented by any one of the following general formula (I), general formula (II), general formula (III) and a salt thereof:
A compound represented by the general formula (I): 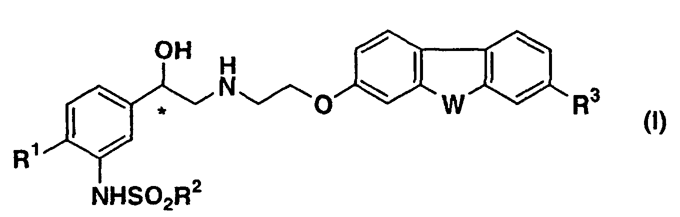 or a salt thereof,
wherein R¹ represents a hydrogen atom, a halogen atom or a hydroxyl group, and R² represents a lower alkyl group or a benzyl group. R³ represents OR, a halogen atom, a trifluoromethyl group, a lower alkyl group, a lower acyl group, NR⁴R^{4'}, a nitro group or a cyano group. Further, R represents a hydrogen atom, a lower alkyl group, a benzyl group or an optionally substituted lower acyl group, and R⁴ and R^{4'} may be the same or different each other and represents a hydrogen atom, a lower alkyl group, a lower acyl group, a benzyl group or SO₂R⁵. R⁵ represents a lower alkyl group or a benzyl group. W represents an oxygen atom, a secondary nitrogen atom (NH) or a sulfur atom.
* means an asymmetric carbon atom,
A compound represented by the general formula (II): 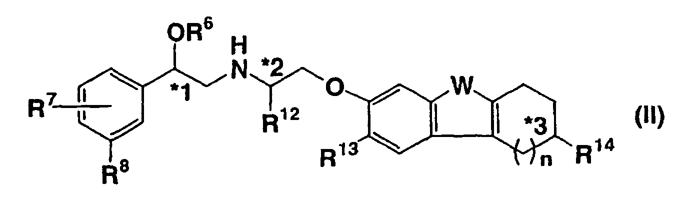 or a salt thereof,
wherein R⁶ represents a hydrogen atom or a methyl group; R⁷ represents a hydrogen atom, a halogen atom, a hydroxyl group, a benzyloxy group, an amino group or a hydroxymethyl group; and R⁸ represents a hydrogen atom, a hydroxymethyl group, NHR⁹, SO₂NR¹⁰R^{10'} or a nitro group. Provided that R⁹ represents a hydrogen atom, a methyl group, SO₂R¹¹, a formyl group or CONHR^{12'}, and R¹¹ represents a lower alkyl group, a benzyl group or NR¹⁰R^{10'}. Further, R¹⁰ and R^{10'} may be the same or different each other and represents a hydrogen atom, a lower alkyl group or a benzyl group. R^{12'} represents a hydrogen atom or a lower alkyl group. Further, R¹² represents a hydrogen atom or a lower alkyl group. n represents 1 or 2, and W represents a secondary nitrogen atom, an oxygen atom or a sulfur atom. When n is 1, one of R¹³ and R¹⁴ represents a hydrogen atom and the other represents a hydrogen atom, an amino group, an acetylamino group or a hydroxyl group. When n represents 2, R¹⁴ represents a hydrogen atom and R¹³ represents a hydrogen atom, an amino group, an acetylamino group or a hydroxyl group.
*1 represents an asymmetric carbon atom, and when neither R¹² nor R¹⁴ is a hydrogen atom.
*2 and *3 mean an asymmetric carbon atom,
A compound represented by the general formula (III): 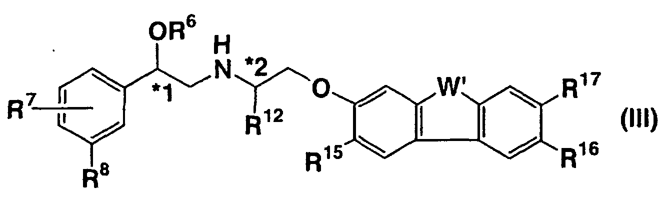
or a salt thereof,
wherein R⁶ represents a hydrogen atom or a methyl group; R⁷ represents a hydrogen atom, a halogen atom, a hydroxyl group, a benzyloxy group, an amino group or a hydroxymethyl group; and R⁸ represents a hydrogen atom, a hydroxymethyl group, NHR⁹, SO₂NR¹⁰R^{10'} or a nitro group. Provided that R⁹ represents a hydrogen atom, a methyl group, SO₂R¹¹, a formyl group or CONHR^{12'}, and R¹¹ represents a lower alkyl group, a benzyl group or NR¹⁰R^{10'}. Further, R¹⁰ and R^{10'} may be the same or different each other and represents a hydrogen atom, a lower alkyl group or a benzyl group. R^{12'} represents a hydrogen atom or a lower alkyl group. Further, R¹² represents a hydrogen atom or a lower alkyl group. W' represents a secondary nitrogen atom, an oxygen atom, a sulfur atom or a methylene group; and when W' is a secondary nitrogen atom, an oxygen atom or a sulfur atom, R¹⁷ represents a hydrogen atom and one of R¹⁵ and R¹⁶ represents a hydrogen atom and the other represents a hydrogen atom, an amino group, an acetylamino group or a hydroxyl group. Further, when W' is a methylene group, R¹⁵ and R¹⁶ each independently represent a hydrogen atom and R¹⁷ represents a hydrogen atom, an amino group, an acetylamino group or a hydroxyl group.
*1 represents an asymmetric carbon atom, and when R¹² is a lower alkyl group.
*2 means an asymmetric carbon atom.

43. The treatment method of claim 41 or 42, which is a treatment method for pollakiuria and urinary incontinence, comprising administration of a therapeutic agent comprising at least an anticholinergic agent and a compound having a β3 agonist activity.

44. The treatment method of claim 41 or 42, which is a treatment method for obesity, comprising administration of a therapeutic agent comprising at least a compound having a β3 agonist activity and one member selected from the group consisting of a monoamine reuptake inhibitor, a lipase inhibitor and a selective serotonin reuptake inhibitor.

45. The treatment method of claim 41 or 42, which is a treatment method for diabetes mellitus, comprising administration of a therapeutic agent comprising at least a compound having a β3 agonist activity and one member selected from the group consisting of insulin, an insulin secretagogue, biguanide, an α-glucosidase inhibitor and an insulin sensitizer.

46. The treatment method of claim 41 or 42, which is a treatment method for hyperlipemia, comprising administration of a therapeutic agent comprising at least a compound having a β3 agonist activity and one member selected from the group consisting of a HMG-CoA reductase inhibitor, an anion exchange resin, a fibric acid derivative and a nicotinic acid derivative.
